# EUROPEAN PATENT SPECIFICATION

(11) **EP 3 408 394 B1**
(45) Date of publication and mention of the grant of the patent: **12.08.2020**
(21) Application number: 17704681.0
(22) Date of filing: 27.01.2017
(51) Int. Cl.: C12N 15/67, C12N 15/82

(54) **MULTIGENE EXPRESSION IN MICROALGAE**
MULTIGENEXPRESSION IN MIKROALGEN
EXPRESSION MULTIGÉNIQUE DANS DES MICRO-ALGUES

(30) Priority: 27.01.2016 EP 16152953
(43) Date of publication of application: 05.12.2018
(73) Proprietor: Total Raffinage Chimie, 92400 Courbevoie (FR)
(72) Inventor: COLLIN, Séverine, 38360 Sassenage (FR); FOURAGE, Laurent, 92150 Suresnes (FR); LAEUFFER, Frédéric, 75002 Paris (FR); BEDHOMME, Mariette, 38450 Vif (FR); MAGNESCHI, Leonardo, Edinburgh EH3 9JH (GB); FINAZZI, Giovanni, 38600 Fontaine (FR); MARECHAL, Eric, 38000 Grenoble (FR)
(74) Representative: De Clercq & Partners
(86) International application number: PCT/EP2017/051723
(87) International publication number: WO 2017/129723

(56) References cited:
- WO-A1-2015/063647
- US-A1- 2010 178 671
- BETH A. RASALA ET AL: "Enhanced Genetic Tools for Engineering Multigene Traits into Green Algae", PLOS ONE, vol. 9, no. 4, 7 April 2014 (2014-04-07), page e94028, XP055281344, DOI: 10.1371/journal.pone.0094028 cited in the application
- MARCO DE GIORGI ET AL: "Co-expression of functional human Heme Oxygenase 1, Ecto-5'-Nucleotidase and ecto-nucleoside triphosphate diphosphohydrolase-1 by "self-cleaving" 2A peptide system", PLASMID, vol. 79, 14 March 2015 (2015-03-14), pages 22-29, XP055281482, US ISSN: 0147-619X, DOI: 10.1016/j.plasmid.2015.03.004
- LAUERSEN KYLE J ET AL: "Targeted expression of nuclear transgenes in Chlamydomonas reinhardtiiwith a versatile, modular vector toolkit", APPLIED MICROBIOLOGY AND BIOTECHNOLOGY, SPRINGER, DE, vol. 99, no. 8, 15 January 2015 (2015-01-15), pages 3491-3503, XP035475457, ISSN: 0175-7598, DOI: 10.1007/S00253-014-6354-7 [retrieved on 2015-01-15]

### SEQUENCE LISTING

<110> TOTAL RAFFINAGE CHIMIE
<120> MULTIGENE EXPRESSION IN MICROALGAE
<130> TOTAL-206-PCT
<150> EP16152953.2
   <151> 27-01-2016
<160> 1
<170> PatentIn version 3.5
<210> 1
   <211> 24
   <212> PRT
   <213> Foot-and-mouth disease virus
<400> 1
   Ala Pro Val Lys Gln Thr Leu Asn Phe Asp Leu Leu Lys Leu Ala Gly
   1               5                   10                  15
   Asp Val Glu Ser Asn Pro Gly Pro
               20

## Description

### FIELD OF THE INVENTION

The present invention is directed to genetic engineering of microalgae, and in particular to engineering microalgae with multiple genes.

### BACKGROUND

Multigene overexpression in eukaryotic microalgae is nowadays limited amongst others by the availability of only a small group of suitable promoters and a limited set of suitable selectable marker genes.

One approach that has been used in microalgae, in particular in *Phaeodactylum tricornutum,* to co-express two genes was the use of the same promoter for the different genes (Hamilton et al., 2014 Metab Eng. 22:3-9). It has however drawbacks for the co-expression of a larger number of genes, namely, that nucleic acid sequences with a large number of identical promoters often stick together and become unstable. Moreover, in general promoters increase the size of the nucleic acid construct, which may then become too large to be introduced in the host cell in a single step. Another strategy for bi-cistronic gene expression was shown in *Chlamydomonas* (Muto et al., 2009 BMC Biotechnology 9:26), wherein the 2 genes were genetically linked to result in a fusion protein that is cleavable by an endogenous protein. Yet another technology that has proven to be useful for multigene expression in microalgae comprises the expression of several proteins from a single open reading frame, wherein each protein is separated by so-called 2A sequences (Ryan et al., 1991 J Gen Virol. 72:2727-32). When the ribosome translates the 2A sequence, it releases the nascent peptide and continues translation of the downstream sequence. As a result, several different, separate proteins can be formed from a single open reading frame. This 2A sequence approach, more particularly using the foot-and-mouth disease virus (FMDV) 2A peptide, was successfully applied in *Chlamydomonas* to overexpress two genes (Rasala et al. 2012 PLoS One 7:e43349). In 2014, the same authors described the use of 2 different 2A peptides, in particular F2A and E2A, in the same construct to genetically engineer *Chlamydomonas* (Rasala et al. 2014 PLoS One 9:e94028). They did engineering with reporter genes only however. They also used the resistance in their multigene expression cassette. In WO 2014026770, the use of 2A sequences for multigene insertion in microalgae species including *Nannochloropsis* and the diatom *Phaeodactylum* is described. It is reported that 2A peptides can be used to express two or more (up to 20 or more) functional proteins from a single mRNA. More than two 2A sequences could be used to increase the number of genes under the control of the same promoter. This increases however the size of the mRNA to be transcribed and could lead to exhaustion of the ribosome. In this case, premature stop in transcription could occur which would result in no synthesis of some proteins downstream. Mulo et al. (2015 Appl Microbiol Biotechnol. 99:3491-3503) disclose a versatile vector toolkit for multiple transgene expression in microalgae.

In view of the above, it is clear that there remains a need in the art for multigene engineering in microalgae.

### SUMMARY OF THE INVENTION

The instant invention aims to provide a system for multigene engineering in microalgae. The inventors have identified particular methods involving the use of self-cleaving viral 2A peptides or 2A-like peptides that allow for efficient multigene overexpression in microalgae.

The present invention is in particular captured by any one or any combination of one or more of the below numbered aspects and embodiments (i) to (xv) wherein:
(i) A multigene expression system for simultaneous expression of at least 6 transgenes, comprising at least two different nucleic acid expression cassettes, wherein each expression cassette comprises a promoter operably linked to three or more transgenes connected to one another by at least one sequence encoding a 2A peptide, and wherein the promoters of the expression cassettes are the same.
(ii) The expression system according to (i), wherein each expression cassette comprises a promoter operably linked to three or more transgenes connected to one another by at least two successive sequences encoding a 2A peptide.
(iii) The expression system according to (i) or (ii), wherein each expression cassette comprises three transgenes.
(iv) The expression system according to any one of (i) to (iii), wherein the 2A peptide is derived from foot-and-mouth disease virus (FMDV 2A or F2A).
(v) The expression system according to any one of (i) to (iv), further comprising one or more nucleic acid expression cassettes comprising a selectable marker gene.
(vi) The expression system according to any one of (i) to (v), wherein the transgenes encode for enzymes involved in a biosynthetic pathway.
(vii) The expression system according to (vi), wherein the transgenes encode enzymes involved in the fatty acid biosynthetic pathway.
(viii) A vector system comprising the expression system according to any one of (i) to (vii), said vector system comprising at least two vectors, wherein each vector comprises one of said at least two nucleic acid expression cassettes comprising a promoter operably linked to three or more transgenes connected to one another by at least one sequence encoding a 2A peptide.
(ix) The vector system according to (viii), wherein each vector further comprises a nucleic acid expression cassette comprising a selectable marker gene.
(x) The vector system according to (viii) or (ix), wherein the vectors are plasmids.
(xi) host cell comprising the expression system according to any one of (i) to (vii) or the vector system according to any one of (viii) to (x).
(xii) The host cell according to (xi), wherein the host cell is a microalga, preferably a diatom such as *Phaeodactylum tricornutum,* or a *Nannochloropsis* species.
(xiii) A method for genetically modifying a host cell with multiple genes comprising the following steps:
   - providing a host cell, and
   - transforming the host cell with at least two different nucleic acid expression cassettes,
      wherein each expression cassette comprises a promoter operably linked to three or more transgenes connected to one another by at least one sequence encoding a 2A peptide, and optionally one or more nucleic acid expression cassettes comprising a selectable marker gene.
(xiv) The method according to (xiii), wherein the at least two nucleic acid expression cassettes and optionally the one or more nucleic acid expression cassettes comprising a selectable marker gene are co-transformed into the host cell.
(xv) The method according to (xiii) or (xiv), further comprising the step of selecting the host cells which have been transformed with said at least two nucleic acid cassettes and said one or more nucleic acid expression cassettes comprising a selectable marker gene by culturing the host cells on a selective medium, wherein the ability of a host cell to be cultured on the selective medium is dependent on the expression of the selectable marker gene.

### BRIEF DESCRIPTION OF THE FIGURES

The teaching of the application is illustrated by the following Figures which are to be considered as illustrative only and do not in any way limit the scope of the claims.

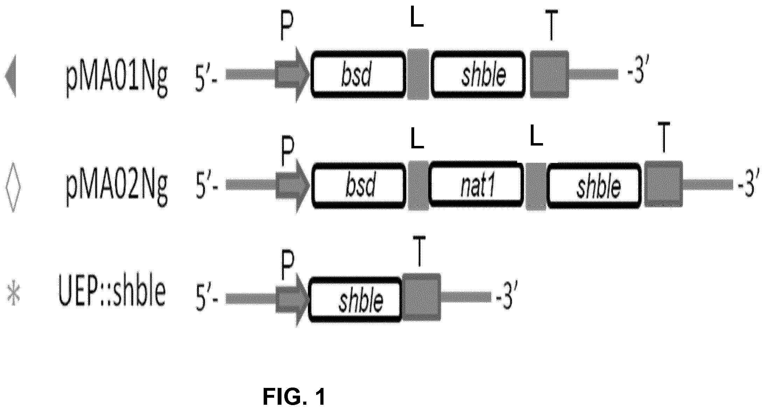

**Figure 1****:** Schematic illustration of constructs for transformation in *Nannochloropsis.* UEP: reference vector comprising only the *shble* gene. pMA01: vector comprising *bsd* and *shble* genes linked with 2A-linker. pMA02: vector comprising *bsd, nat1* and *shbe* genes linked with 2A-linker. P= promoter; T= terminator; L=linker.

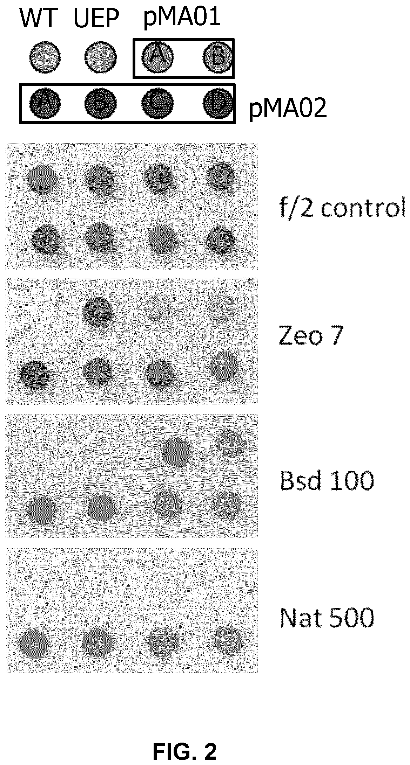

**Figure 2****:** Spot test on f/2 agar plate without (f/2 control) or with 7 µg/ml zeocin (Zeo 7), 100 µg/ml blasticidin (Bsd 100), or 500 µg/ml nourseothricin (Nat 500) with WT *Nannochloropsis* (WT), or *Nannochloropsis* transformed with UEP vector, pMA01 vector, or pMA02 vector.

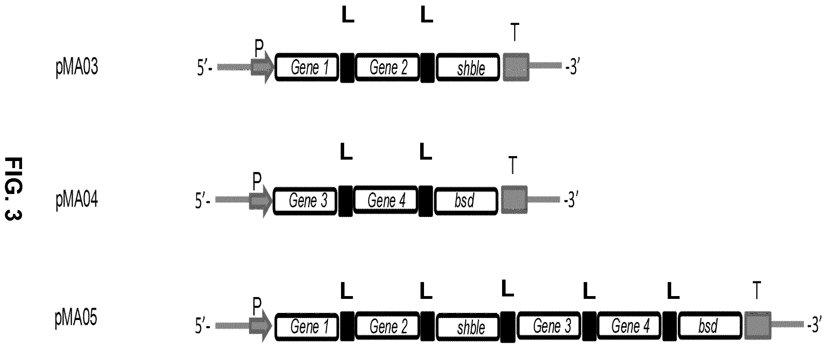

**Figure 3****:** Schematic illustration of constructs for co-transformation (pMA03+pMA04) or transformation (pMA05) in *Nannochloropsis.* pMA03: construct comprising *gene 1, gene* 2 and *shble* gene linked with 2A-linker. pMA04: construct comprising *gene 3, gene 4* and *bsd* gene linked with 2A-linker. pMA05: construct comprising *gene 1, gene 2, shble* gene, *gene 3, gene 4* and *bsd* gene linked with 2A-linker. P= promoter; T= terminator; L=linker.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. Where reference is made to embodiments as comprising certain elements or steps, this encompasses also embodiments which consist essentially of the recited elements or steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.

Standard reference work setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates).

The terms **"polynucleotide"** and **"nucleic acid"** are used interchangeably herein and generally refer to a polymer of any length composed essentially of nucleotides, e.g., deoxyribonucleotides and/or ribonucleotides. Nucleic acids can comprise purine and/or pyrimidine bases, and/or other natural, chemically or biochemically modified (e.g., methylated), non-natural, or derivatised nucleotide bases. The backbone of nucleic acids can comprise sugars and phosphate groups, as can typically be found in RNA or DNA, and/or one or more modified or substituted (such as, 2'-O-alkylated, e.g., 2'-O-methylated or 2'-O-ethylated; or 2'-O,4'-C-alkynelated, e.g., 2'-O,4'-C-ethylated) sugars or one or more modified or substituted phosphate groups. For example, backbone analogues in nucleic acids may include phosphodiester, phosphorothioate, phosphorodithioate, methylphosphonate, phosphoramidate, alkyl phosphotriester, sulfamate, 3'-thioacetal, methylene (methylimino), 3'-N-carbamate, morpholino carbamate, and peptide nucleic acids (PNAs). The term nucleic acid further specifically encompasses DNA, RNA and DNA/RNA hybrid molecules, specifically including hnRNA, pre-mRNA, mRNA, cDNA, genomic DNA, gene, amplification products, oligonucleotides, and synthetic (e.g. chemically synthesised) DNA, RNA or DNA/RNA hybrids. The terms "ribonucleic acid" and "RNA" as used herein mean a polymer of any length composed of ribonucleotides. The terms "deoxyribonucleic acid" and "DNA" as used herein mean a polymer of any length composed of deoxyribonucleotides. The term "DNA/RNA hybrid" as used herein mean a polymer of any length composed of one or more deoxyribonucleotides and one or more ribonucleotides. A nucleic acid can be naturally occurring, e.g., present in or isolated from nature, can be recombinant, i.e., produced by recombinant DNA technology, and/or can be, partly or entirely, chemically or biochemically synthesized. A nucleic acid can be double-stranded, partly double stranded, or single-stranded. Where single-stranded, the nucleic acid can be the sense strand or the antisense strand. In addition, nucleic acid can be circular or linear.

As used herein, the term **"nucleic acid expression cassette"** refers to nucleic acid molecules that include one or more transcriptional control elements (such as, but not limited to promoters, enhancers, polyadenylation sequences, and introns) that direct expression of a (trans)gene(s) to which they are operably linked.

The term **"operably linked"** as used herein refers to the arrangement of various nucleic acid molecule elements relative to each such that the elements are functionally connected and are able to interact with each other in the context of gene expression. Such elements may include, without limitation, a promoter, an enhancer, a polyadenylation sequence, one or more introns, and a coding sequence of a gene of interest to be expressed (e.g., the (trans)gene). The nucleic acid sequence elements, when properly oriented or operably linked, act together to ensure or modulate expression of the coding sequence. By modulate is meant increasing, decreasing, or maintaining the level of activity of a particular element. The position of each element relative to other elements may be expressed in terms of the 5' terminus and the 3' terminus of each element, and the distance between any particular elements may be referenced by the number of intervening nucleotides, or base pairs, between the elements.

The term **"transgene"** or **"(trans)gene"** as used herein refers to particular nucleic acid sequences encoding a polypeptide or a portion of a polypeptide to be expressed in a host cell into which the nucleic acid sequence is introduced. How the nucleic acid sequence is introduced into a host cell is not essential, it may for instance be through integration in the genome or as an episomal plasmid. The term "transgene" is meant to include (1) a nucleic acid sequence that is not naturally found in the host cell (i.e., a heterologous nucleic acid sequence); (2) a nucleic acid sequence that is a mutant form of a nucleic acid sequence naturally found in the host cell into which it has been introduced ; (3) a nucleic acid sequence that serves to add additional copies of the same (i.e., homologous) or a similar nucleic acid sequence naturally occurring in the host cell into which it has been introduced; or (4) a silent naturally occurring or homologous nucleic acid sequence whose expression is induced in the host cell into which it has been introduced. Accordingly, a "transgene" is characterized by the fact that it does not naturally occur in the same location in the host cell. By "mutant form" is meant a nucleic acid sequence that contains one or more nucleotides that are different from the wild-type or naturally occurring sequence, i.e., the mutant nucleic acid sequence contains one or more nucleotide substitutions, deletions, and/or insertions.

The term **"cistron"** generally refers to nucleic acid sequences encoding a gene product (such as a protein or RNA molecule) and including upstream and downstream transcriptional control elements. As used herein, the term **"multicistron"** refers to multiple nucleic acid sequences encoding gene products and including upstream and downstream transcriptional control elements. Typical for a multicistron is that the multiple coding sequences are under the control of a single promoter. The term "tricistron" as used herein specifically refers to a multicistron comprising three coding sequences.

The term **"2A peptide"** (also referred to as CHYSEL or cis-acting hydrolase element) refers to a viral sequence of about 18 to 22 amino acids which upon translation, mediates rapid intramolecular (cis) cleavage of a protein or polypeptide comprising the peptide to yield discrete mature proteins or polypeptides; this "cleavage" does not require any additional factors like proteases. The term "2A peptide" as used herein also includes any modification of the sequence of the 2A peptide which may improve, increase or have a neutral effect regarding the functionality of the 2A peptide.

As used in the application, the term **"promoter"** refers to a nucleic acid sequence capable of binding RNA polymerase and that initiates the transcription of one or more nucleic acid coding sequences to which it is operably linked (e.g., a transgene). A promoter is usually located near the transcription start site of a gene on the same strand and upstream on the nucleotide coding sequence (5' in the sense strand). A promoter may function alone to regulate transcription or may be further regulated by one or more regulatory sequences (e.g. enhancers or silencers).

The term **"transcription termination sequence"** encompasses a control sequence at the end of a transcriptional unit, which signals 3' processing and termination of transcription.

As used herein, the term **"selectable marker gene"** includes any gene, which confers a phenotype on a host cell in which it is expressed to facilitate the identification and/or selection of host cells which are transfected or transformed with a transgene.

By **"vector"** is meant a polynucleotide molecule, preferably a DNA molecule derived, for example, from a plasmid, bacteriophage, or plant virus, into which a polynucleotide can be inserted or cloned. A vector preferably contains one or more unique restriction sites and can be capable of autonomous replication in a defined host cell, or can be integrated within the genome of the defined host such that the cloned sequence is reproducible. The choice of the vector will typically depend on the compatibility of the vector with the host cell into which the vector is to be introduced.

As used herein, the term **"host cell"** refers to those cells used for transformation, i.e. for expression of transgenes. A host cell may be an isolated cell or a cell line grown in culture, or a cell which resides in a living tissue or organism. In the context of the present invention, the host cells are preferably cells that are capable of growth in culture.

The term **"microalgae"** as used herein refers to microscopic algae. "Microalgae" encompass, without limitation, organisms within (i) several eukaryotic phyla, including the Rhodophyta (red algae), Chlorophyta (green algae), Dinoflagellata, Haptophyta, (ii) several classes from the eukaryotic phylum Heterokontophyta which includes, without limitation, the classes Bacillariophycea (diatoms), Eustigmatophycea, Phaeophyceae (brown algae), Xanthophyceae (yellow-green algae) and Chrysophyceae (golden algae), and (iii) the prokaryotic phylum Cyanobacteria (blue-green algae). The term "microalgae" includes for example genera selected from: *Achnanthes, Amphora, Anabaena, Anikstrodesmis, Arachnoidiscusm, Aster, Botryococcus, Chaetoceros, Chlamydomonas, Chlorella, Chlorococcum, Chorethron, Cocconeis, Coscinodiscus, Crypthecodinium, Cyclotella, Cylindrotheca, Desmodesmus, Dunaliella, Emiliana, Euglena, Fistulifera, Fragilariopsis, Gyrosigma, Hematococcus, Isochrysis, Lampriscus, Monochrysis, Monoraphidium, Nannochloris, Nannochloropsis, Navicula, Neochloris, Nephrochloris, Nephroselmis, Nitzschia, Nodularia, Nostoc, Odontella, Oochromonas, Oocystis, Oscillartoria, Pavlova, Phaeodactylum, Playtmonas, Pleurochrysis, Porhyra, Pseudoanabaena, Pyramimonas, Scenedesmus, Schyzochitrium, Stichococcus, Synechococcus, Synechocystis, Tetraselmis, Thalassiosira,* and *Trichodesmium.*

The term **"transformation"** means introducing an exogenous nucleic acid into an organism so that the nucleic acid is replicable, either as an extrachromosomal element or by chromosomal integration.

The present application generally relates to multigene engineering, and more particularly to multigene overexpression, in microalgae.

More particularly, the application provides expression systems for multigene overexpression in microalgae and vector systems comprising said expression systems, host cells transformed with said expression systems or comprising said vector systems, methods for producing these host cells, as well as their use for biosynthetic processes, as defined in the claims. The inventors have found that the use of at least two multicistronic expression cassettes such as tricistronic expression cassettes, wherein the transgenes are connected to one another by at least one sequence encoding a 2A peptide, and preferably driven by the same promoter, allows for efficient expression of multiple genes in microalgae, overcoming the existing limitations for multigene overexpression in these organisms. The different aspects of the invention are detailed herein below.

### Expression system

The expression systems provided in the context of the present invention comprise at least two different multicistronic such as tricistronic expression cassettes. Accordingly, the expression system provided herein comprises at least a first nucleic acid expression cassette and a second nucleic acid expression cassette, wherein said first and said second expression cassettes are not copies of each other, and wherein each expression cassette comprises a promoter operably linked to three or more transgenes connected to one another by at least one sequence encoding a 2A peptide.

The multicistronic expression cassettes envisaged herein comprise three or more, such as three, four, five, six or more, transgenes of interest, which are under the control of a single promoter, and which are connected to one another by at least one sequence encoding a 2A peptide. In certain embodiments, the multicistronic expression cassettes are tricistronic expression cassettes which comprise three transgenes of interest under the control of a single promoter, wherein said three transgenes are connected to one another by at least one sequence encoding a 2A peptide.

The expression system of the present invention is characterized in that it comprises at least two of these multicistronic or tricistronic expression cassettes. The two or more multicistronic or tricistronic expression cassettes are different and thus not copies of each other. The two or more multicistronic expression cassettes may comprise an equal number of transgenes, or the number of transgenes comprised in the two or more expression cassettes may be different.

Each of these multicistronic or tricistronic expression cassettes is operably linked to a promoter and the promoter for the different multicistronic or tricistronic expression cassettes may be the same or different. According to the invention, the promoters of the multicistronic or tricistronic expression cassettes of the expression system of the invention are the same. This is advantageous for expression in microalgae, for which only a limited list of suitable promoters is available. In this way a significant number of genes (at least 6) can be expressed simultaneously without the disadvantages of the use of multiple promoters which increase the size of the construct and may further compromise the stability of the construct where the use of identical promoters is envisaged.

The 2A peptide used in the expression systems envisaged herein may be derived from a mammalian virus such as foot and mouth disease virus (FMDV), cardiovirus encephalomyocarditis virus (EMCV), Theiler's murine encephalitis (TMEV), bovine type C rotavirus (BRCV), Porcine type C rotavirus (PRCV), Human type C rotavirus (HRCV), equine rhinitis A virus (ERAV), equine rhinitis B virus (ERBV) and porcine teschovirus-1 (PTV-1; formerly porcine enteovirus-1). The 2A peptide may also be derived from an insect virus selected from the group comprising *Thoseaasigna virus* (TaV), infectious flacherie virus (IFV), *Drosophila* C virus (DCV) acute bee paralysis virus (ABPV) and cricket paralysis virus (CrPV). The 2A peptide may also be derived from *Trypansoma* spp., including *T. brucei* (TSR1) and *T. cruzi* (AP endonuclease) as described in Ryan et al. (2002, in Molecular Biology of Picornaviruses Ed. Semler and Wimmer, p. 213-223) or from Ljungan virus (174F, 145SL, 87-012, M1146). Preferably, the 2A peptide is derived from FMDV or EMCV. In embodiments, the sequence encoding the 2A peptide is the FMDV 2A sequence (APVKQTLNFDLLKLAGDVESNPGP, SEQ ID NO:1).

The transgenes in the multicistronic expression cassettes such as the tricistronic expression cassettes envisaged herein are connected to one another by at least one sequence encoding a 2A peptide. In certain embodiments, the transgenes are connected to one another by at least two, such as two, three, four, five or more, successive sequences encoding a 2A peptide. The use of two or more successive 2A increases the likelihood of cleavage of the transgene products.

In embodiments, the successive 2A sequences are the same. In other embodiments, at least one 2A sequence of the successive 2A sequences is different. The use of different 2A sequences reduces the likelihood of homologous recombination events.

In embodiments, the 2A peptides in each of the two or more multicistronic expression cassettes are the same. In other embodiments, the 2A peptides present in the two or more multicistronic expression cassette are different. In particular embodiments, the 2A peptides used within one multicistronic expression cassette are the same, but are different from the 2A peptides used in the other of the two or more multicistronic expression cassettes. In particular embodiments, the 2A peptides used within one multicistronic expression cassette are different from each other but the same in each of the two or more multicistronic expression cassettes.

The transgenes that are envisaged for transformation using the expression system of the present invention are not critical. Indeed the present system can allow multigene (over)expression in microalgae independent of the nature of the transgene.

In particular embodiments, the transgenes encode enzymes involved in biosynthetic pathways. Indeed, multigene transformation is of particular interest in the context of introducing biosynthetic pathways into a host organism. The expression systems of the present invention allow for the simultaneous and co-localized expression of several genes relating to a biosynthetic pathway. The co-expression of different enzymes involved in subsequent steps of a biosynthetic pathway significantly furthers their efficiency.

For example, the transgenes in the multicistronic or tricistronic expression cassettes encode enzymes involved in the fatty acid biosynthetic pathway (also referred to as fatty acid enzymes herein). These multicistronic or tricistronic expression cassettes are of particular interest for the recombinant production of fatty acids, e.g. through the (over)expression of said multicistronic or tricistronic expression cassettes in a recombinant host cell, as detailed below. Exemplary genes involved in fatty acid synthesis include, without limitation genes encoding pyruvate dehydrogenase complex (PDH), acetyl-CoA carboxylase (ACCase), malonyl-CoA:ACP transacylase (MAT), 3-ketoacyl-ACP synthase (KAS), 3-ketoacyl-ACP reductase (KAR), 3-hydroxyacyl-ACP dehydratase (HD), enoyl-ACP reductase (ENR), fatty acyl-ACP thioesterase (FAT), glycerol-3-phosphateacyltransferase (GPAT), lyso-phosphatidicacidacyltransferase (LPAAT), lyso-phosphatidylcholineacyltransferase (LPAT), diacylglycerolacyltransferase (DAGAT), or glycerol-3-phosphate dehydrogenase (G3PDH), as described e.g. in Radakovits et al. (2010 Eukaryotic Cell 486:501).

Promoters envisaged in the context of the present invention will be determined by its ability to direct expression in the host cell of interest. Preferably, the promoter is a promoter from microalgae. Exemplary promoters include, without limitation, those from *Chlamydomonas reinhardtii,* and from *Chlorella* species including *Chlorella vulgaris, Nannochloropsis sp, Phaeodactylum tricornutum, Thalassiosira sp, Dunaliella salina* and *Haematococcus pluvialis.* Non-limiting examples of suitable promoters are the Hsp70A promoter, the RbcS2 promoter and the beta-2-tubulin (TUB2) promoter from *Chlamydomonas reinhardtii,* the fucoxanthin chlorophyll a/b-binding protein (fcp) promoters, Histone 4 (H4) promoter from *Phaeodactylum tricornutum,* the Nitrate reductase (NR) promoter from *Thalassiosira,* and ubiquitin extension protein (UEP) from *Nannochloropsis* sp. In embodiments, the promoter in the multicistronic or tricistrionic expression cassettes envisaged herein is the Histone 4 (H4) promoter from *Phaeodactylum tricornutum or* ubiquitin extension protein (UEP) from *Nannochloropsis gaditana.*

Other sequences may be incorporated in the multicistronic or tricistronic expression cassettes for use in the invention. More particularly the inclusion of sequences which further increase or stabilize the expression of the transgene products (e.g. introns and/or a transcription termination sequence) is envisaged.

In particular embodiments, the multicistronic or tricistronic expression cassettes further comprise a transcription termination sequence. Any polyadenylation signal that directs the synthesis of a polyA tail is useful in the multicistronic or tricistronic expression cassettes described herein, examples of those are well known to one of skill in the art. Exemplary polyadenylation signals include, but are not limited to, the polyadenylation signal derived from the Simian virus 40 (SV40) late gene, and the bovine growth hormone (BGH) polyadenylation signal, or the terminator region of the fucoxanthin chlorophyll a/b-binding protein (*fcp*) gene, such as the fcpA terminator. In embodiments, the fcpA terminator is used in the multicistronic or tricistronic expression cassettes envisaged herein.

Preferably, the expression systems envisaged herein comprise a selectable marker gene. Said selectable marker gene is preferably not comprised in the multicistronic or tricistronic expression cassettes of the expression system (i.e. the transgenes of the multicistronic or tricistronic expression cassettes do not encode for a selectable marker), but in a separate nucleic acid expression cassette. Accordingly, in embodiments, the expression systems envisaged herein further comprise one or more nucleic acid expression cassettes comprising a selectable marker gene. Expression of the selectable marker gene(s) may indicate that the host cell has been transformed with the multicistronic or tricistronic expression cassettes and hence, allows for selecting transformed host cells. The selectable marker cassette typically further includes a promoter and transcription terminator sequence, operatively linked to the selectable marker gene, and which are operable in the host cell of choice.

Suitable markers may be selected from markers that confer antibiotic resistance, herbicid resistance, visual markers, or markers that complement auxotrophic deficiencies of a host cell. For example, the selection marker may confer resistance to an antibiotic such as hygromycin B (such as the *hph* gene), zeocin/phleomycin (such as the *ble* gene), kanamycin or G418 (such as the *nptlI* or *aphVII*I genes), spectinomycin (such as the *aadA* gene), neomycin (such as the *aphVIII* gene), blasticidin (such as the *bsd* gene), nourseothricin (such as the *natR* gene), puromycin (such as *pac* gene) and paromomycin (such as the *aphVIII* gene). In other examples, the selection marker may confer resistance to a herbicide such as glyphosate (such as GAT gene), oxyfluorfen (such as protox/PPO gene) and norflurazon (such as PDS gene). Visual markers may also be used and include for example beta-glucuronidase (GUS), luciferase and fluorescent proteins such as Green Fluorescent Protein (GFP), Yellow Fluorescent protein, etc. Two prominent examples of auxotrophic deficiencies are the amino acid leucine deficiency (e.g. LEU2 gene) or uracil deficiency (e.g. URA3 gene). Cells that are orotidine-5'-phosphate decarboxylase negative (ura3-) cannot grow on media lacking uracil. Thus a functional URA3 gene can be used as a selection marker on a host cell having a uracil deficiency, and successful transformants can be selected on a medium lacking uracil. Only cells transformed with the functional URA3 gene are able to synthesize uracil and grow on such medium. If the wild-type strain does not have a uracil deficiency, an auxotrophic mutant having the deficiency must be made in order to use URA3 as a selection marker for the strain. Methods for accomplishing this are well known in the art.

### Vector system

The expression cassettes envisaged herein may be used as such, or typically, they may be part of (i.e. introduced into) a nucleic acid vector. The at least two multicistronic or tricistronic expression cassettes of the expression system disclosed herein may be located on the same vector or on different vectors. The present invention particularly envisages a vector system comprising at least two vectors, wherein each vector comprises only one of said at least two multicistronic or tricistronic expression cassettes. The vectors of the vector system envisaged herein are different. In embodiments, the vectors disclosed herein further comprise an expression cassette comprising a selectable marker gene, such as an antibiotic resistance cassette.

The vectors disclosed herein may further include an origin of replication that is required for maintenance and/or replication in a specific cell type. One example is when a vector is required to be maintained in a host cell as an episomal genetic element (e.g. plasmid or cosmid molecule). Exemplary origins of replication include, but are not limited to the f1-ori, colE1 ori, and Gram+ bacteria origins of replication.

The vectors taught herein may further contain restriction sites of various types for linearization or fragmentation.

Numerous vectors are known to practitioners skilled in the art and any such vector may be used. Selection of an appropriate vector is a matter of choice. The vector may be a non-viral or viral vector. Non-viral vectors include but are not limited to plasmids, cationic lipids, liposomes, nanoparticles, PEG, PEI, etc. Viral vectors are derived from viruses including but not limited to: retrovirus, lentivirus, adeno- associated virus, adenovirus, herpesvirus, hepatitis virus or the like. Preferred vectors for this invention are vectors developed for algae such as the vectors commonly known by the skilled person as pPha-T1, pPha-T1-HSP, pPha-T1-TUB and pPhaT1-UEP.

Construction of the vectors described herein containing or including the multicistronic or tricistronic expression cassettes, and optionally the selectable marker cassettes, and one or more of the above listed components employs standard ligation techniques. For example, isolated plasmids may be cleaved, tailored, and re-ligated in the form desired to generate the plasmids required.

### Host cells and methods for making same

A further aspect of the present invention relates to a host cell comprising an expression system or a vector system according to the invention, which host cells are genetically modified with multiple (trans)genes.

The selection of the host cell may be determined by the envisaged application. Particular examples of host cells which may be used in accordance with the present invention are microalgae. Non-limiting examples of microalgae are *Chlamydomonas reinhardtii* strains, *Chlorella* species including *Chlorella vulgaris, Chlorella sorokiniana* and *Chlorella (Auxenochlorella) protothecoides, Dunaliella salina, Haematococcus pluvialis, Ostreococcus tauri, Nannochloropsis* species such as *Nannochloropsis gaditana, Scenedesmus* species, and diatoms such as *Phaeodactylum* species, e.g. *Phaeodactylum tricornutum.* More preferably, the microalga is a *Nannochloropsis* species or a diatom such as *Phaeodactylum tricornutum.*

The microalgae may be for example, but without limitation, microalgae growing in photoautotrophic, mixotrophic or heterotrophic conditions. Most microalgae are photoautotrophs, i.e. their growth is strictly dependent on the generation of photosynthetically-derived energy. Their cultivation hence requires a relatively controlled environment with a large input of light energy. For certain industrial applications, it is advantageous to use heterotrophic microalgae, which can be grown in conventional fermenters. Accordingly, in embodiments the microalgae have been metabolically engineered to grow heterotrophically (i.e. to utilize exogenous organic compounds (such as glucose, acetate, etc.) as an energy or carbon source). A method for metabolically engineering microalgae to grow heterotrophically has been described in US Patent No. 7,939,710, which is specifically incorporated by reference herein. In particular embodiments, the microalgae are further genetically engineered to comprise a recombinant nucleic acid encoding a glucose transporter, preferably a glucose transporter selected from the group consisting of Glut 1 (human erythrocyte glucose transporter 1) and Hup1 (Chlorella HUP1 Monosaccharide-H+ Symporter). The glucose transporters facilitate the uptake of glucose by the host cell, allowing the cells to metabolize exogenous organic carbon and to grow independent of light. This is particularly advantageous for obligate phototrophic microalgae. Lists of phototrophs may be found in a review by Droop (1974. Heterotrophy of Carbon. In Algal Physiology and Biochemistry, Botanical Monographs, 10:530-559, ed. Stewart, University of California Press, Berkeley), and include, for example but without limitation, organisms of the phyla Cyanophyta (Blue-green algae), including the species *Spirulina* and *Anabaena;* Chlorophyta (Green algae), including the species *Dunaliella, Chlamydomonas,* and *Heamatococcus;* Rhodophyta (Red algae), including the species *Porphyridium, Porphyra, Euchema,* and *Graciliaria;* Phaeophyta (Brown algae), including the species, *Macrocystis, Laminaria, Undaria,* and *Fucus;* Baccilariophyta (Diatoms), including the species *Nitzschia, Navicula, Thalassiosira,* and *Phaeodactylum;* Dinophyta (Dinoflagellates), including the species *Gonyaulax;* Chrysophyta (Golden algae), including the species Irsochrysis and *Nannochloropsis;* Cryptophyla, including the species *Cryptomonas;* and Euglenophyta, including the species *Euglena.*

Also provided herein are methods for obtaining a genetically engineered host cell as described herein, which method may comprise transforming, preferably co-transforming, a host cell with the at least two multicistronic or tricistronic expression cassettes or the at least two vectors each comprising one of said at least two multicistronic or tricistronic expression cassettes, as taught herein above. The method may further comprise the step of selecting the cells which have taken up the exogenous nucleic acids. In embodiments wherein the host cells are co-transformed with the at least two vectors of the vector system envisaged herein, said at least two vectors preferably comprise a different selectable marker gene.

Methods used herein for transformation of the host cells are well known to a skilled person. For example, electroporation and/or chemical (such as calcium chloride- or lithium acetate-based) transformation methods or *Agrobacterium tumefaciens*-mediated transformation methods as known in the art can be used.

The multicistronic or tricistronic expression cassettes or vectors disclosed herein may either be integrated into the genome of the host cell or they may be maintained in some form (such as a plasmid) extrachromosomally. A stably transformed host cell is one in which the exogenous nucleic acid has become integrated into a chromosome so that it is inherited by daughter cells through chromosome replication.

Successful transformants can be selected for in known manner, e.g. by taking advantage of the attributes contributed by the marker gene, or by other characteristics resulting from the introduced coding sequences (such as ability to produce fatty acids). Screening can also be performed by PCR or Southern analysis to confirm that the desired insertions have taken place, to confirm copy number and to identify the point of integration of coding sequences into the host genome.

### Producing fatty acids using recombinant host cells

As detailed above, in particular embodiments, it is envisaged to introduce the expression systems of the present invention in the context of biosynthesis, such as fatty acid production. Accordingly, the present invention also relates to the use of an expression system, a vector system or a host cell according to the invention, for biosynthesis, such as the industrial production of fatty acids.

Further disclosed herein are methods for the production of fatty acids, which method comprises providing a genetically engineered host cell wherein enzymes involved in fatty acid biosynthesis have been introduced using the multigene expression systems as described above and culturing said genetically engineered host cell in a culture medium so as to allow the production of fatty acids. More particularly, the host cell is cultured under conditions suitable to ensure expression of the multicistronic or tricistronic expression cassettes, which expression cassettes comprise transgenes encoding enzymes involved in the fatty acid biosynthetic pathway envisaged herein.

In particular the host cells ensure a rate of fatty acid production which is sufficiently high to be industrially valuable. Indeed, in particular, as a result of the coordinated expression of the different enzymes involved, the recombinant host cells disclosed herein are capable of ensuring a high yield at limited production costs. The recombinant host cells are cultured under conditions suitable for the production of fatty acids by the host cells. More particularly this implies "conditions sufficient to allow expression" of the multicistronic or tricistronic expression cassettes (comprising transgenes encoding fatty acid enzymes), which means any condition that allows a host cell to (over)produce a fatty acid enzyme as described herein. Suitable conditions include, for example, fermentation conditions. Fermentation conditions can comprise many parameters, such as temperature ranges, levels of aeration, and media composition. Each of these conditions, individually and in combination, allows the host cell to grow. To determine if conditions are sufficient to allow (over)expression, a host cell can be cultured, for example, for about 4, 8, 12, 18, 24, 36, or 48 hours. During and/or after culturing, samples can be obtained and analyzed to determine if the conditions allow (over)expression. For example, the host cells in the sample or the culture medium in which the host cells were grown can be tested for the presence of a desired product (e.g. a fatty acid). When testing for the presence of a desired product, assays, such as, but not limited to, sodium dodecyl sulfate polyacrylamide gel electrophoresis (SDS-PAGE), TLC, HPLC, GC/FID, GC/MS, LC/MS, MS, can be used.

Exemplary culture media include broths or gels. The host cells may be grown in a culture medium comprising a carbon source to be used for growth of the host cell. Exemplary carbon sources include carbohydrates, such as glucose, fructose, cellulose, or the like, that can be directly metabolized by the host cell. In addition, enzymes can be added to the culture medium to facilitate the mobilization (e.g., the depolymerization of starch or cellulose to fermentable sugars) and subsequent metabolism of the carbon source. A culture medium may optionally contain further nutrients as required by the particular strain, including inorganic nitrogen sources such as ammonia or ammonium salts, and the like, and minerals and the like. In particular embodiments, wherein phototrophic microalgae are used as host cells, the method for the production of fatty acids may comprise providing microalgae genetically engineered to produce fatty acids as taught herein, and culturing said microalgae in photobioreactors or an open pond system using CO₂ and sunlight as feedstock.

Other growth conditions, such as temperature, cell density, and the like are generally selected to provide an economical process. Temperatures during each of the growth phase and the production phase may range from above the freezing temperature of the medium to about 50°C.

The culturing step of the methods of the invention may be conducted aerobically, anaerobically, or substantially anaerobically. Briefly, anaerobic conditions refer to an environment devoid of oxygen. Substantially anaerobic conditions include, for example, a culture, batch fermentation or continuous fermentation such that the dissolved oxygen concentration in the medium remains between 0 and 10% of saturation. Substantially anaerobic conditions also includes growing or resting cells in liquid medium or on solid agar inside a sealed chamber maintained with an atmosphere of less than 1% oxygen. The percent of oxygen can be maintained by, for example, sparging the culture with an N₂/CO₂ mixture or other suitable non-oxygen gas or gasses.

The cultivation step of the methods described herein can be conducted continuously, batch-wise, or some combination thereof.

Further disclosed are methods for producing fatty acids, which, in addition to the steps detailed above, further comprise the step of recovering the fatty acids from the host cell or the culture medium. Suitable purification can be carried out by methods known to the person skilled in the art such as by using lysis methods, extraction, ion exchange resins, electrodialysis, nanofiltration, etc.

Accordingly, methods are disclosed for the production of fatty acids which methods comprise the steps of:
(i) providing a genetically engineered host cell transformed using a multigene expression system which ensures expression of enzymes involved in fatty acid biosynthesis as described herein above;
(ii) culturing the host cells under conditions suitable for the production of fatty acids, and
(iii) recovering the fatty acids from the host cell or the culture medium.

The invention will be further understood with reference to the following non-limiting examples.

### EXAMPLES

The practice of the present invention will employ, unless otherwise indicated, conventional techniques used in recombinant DNA technology, molecular biology, biological testing, and the like, which are within the skill of the art. Such techniques are explained fully in the literature.

### Example 1: Multigene expression in Phaeodactylum tricornutum

### Materials and methods

DNA expression cassettes were constructed which comprise the genes bsd (from *Aspergillus terreus), nat1 (*from *Streptomyces noursei)* and *shble (*gene from pUT58 *(*Drocourt et al.,1990 Nucleic Acids Research 18: 4009), which confer resistance to respectively, the antibiotics blasticidin, nourseothricin and zeocin. The genes were separated by a nucleotide sequence encoding the F2A peptide (F2A; APVKQTLNFDLLKLAGDVESNPGP, SEQ ID NO:1), and were under the control of the histone 4 (H4p) promoter of *Phaeodactylum tricornutum.* The fucoxanthin chlorophyll a binding protein (fcpA) terminator was further integrated behind the tricistronic construct.

The expression cassettes were ligated into pCT2 vectors, each vector comprising only one tricistronic expression cassette, and further comprising an antibiotic resistance cassette.

*Phaeodactylum tricornutum* cells were transformed using an adapted NEPA21 electroporation protocol as described by Miyahara et al. (2013 Biosci Biotechnol Biochem. 77(4):874-876) with the constructed vectors and allowed to randomly insert the expression cassettes in their genome. Briefly, 1.10⁷ cells were collected by centrifugation at 1500xg, washed twice with 1 ml 0,77 M mannitol, re-suspended in 150 µl 0,77 M mannitol and transferred to 0,2 cm electroporation cuvettes. 4 µg of vectors were electroporated. Cells were transferred to 50 ml tubes in 5 ml ESAW medium (Harrison et al. 1980 J. Phycol. 16:28-35) and allowed to recover for 20 hours at 20°C in a 12:12 dark:light regimen while shaking at 100 RPM. Cells were collected and plated onto 100 µg/ml zeocin containing agar plates and incubated one month under the same light and temperature conditions. Clones resistant to zeocin were then re-plated on the 2 other antibiotics.

### Results

The clones were resistant to the 3 antibiotics, which in all likelihood is due to the expression of all 3 genes linked by the F2A sequences.

### Example 2: Multigene expression in Nannochloropsis

### Materials and methods

The following DNA constructs were prepared (Fig. 1):
UEP construct: comprising a promoter operably linked to the *shble* gene that confers resistance to the antibiotic zeocin, and a terminator;
pMA01 construct: comprising a promoter operably linked to the *bsd* gene that confers resistance to the antibiotic blasticidin and the *shBle* gene fused to a His-tag, and a terminator; and
pMA02 construct: comprising a promoter operably linked to the bsd gene, the *nat1* gene that confers resistance to the antibiotic nourseothricin, and the *shBle* gene fused to a His-tag, and a terminator.

The F2A sequence (APVKQTLNFDLLKLAGDVESNPGP; SEQ ID NO:1) was used as linker between the antibiotic resistance genes in the pMA01 and pMA02 constructs.

The constructs were transformed in *Nannochloropsis gaditana 526.* Briefly, 1.10⁸ cells were collected by centrifugation at 3500xg for 13 min, washed twice in 1 ml 375 mM D-sorbitol and re-suspended in 100 µl D-sorbitol. 2µg of linearized DNA was added to the cells, kept on ice for 15 min and electroporated into 0,2 cm cuvettes, using 2400 V, 500 Ohms, 50 µF. After electroporation, cells were transferred to 5 ml of f/2 medium and incubated for 24 hours in constant light, at 100 RPM and 20°C. Cells were then pelleted (3500xg for 5min), and plated onto selective 1% agar plate containing 7 µg/ml zeocin (Zeo 7), 100 µg/ml blasticidin (Bsd 100), or 500 µg/ml nourseothricin (Nat 500), and incubated under the same conditions.

### Results

The pMA01 and pMA02 transformants were resistant to zeocin and blasticidin, and zeocin, blasticidin and nourseothricin, respectively (Fig. 2). These data show that the 2A sequence-separated resistance genes were functional in *Nannochloropsis.*

### Example 3: Co-expression of 2 multigene constructs in Nannochloropsis

### Materials and methods

The following DNA constructs are prepared (Fig.3):
pMA03 construct: comprising a promoter operably linked to *gene 1, gene 2* and the *shBle* gene that confers resistance to the antibiotic zeocin, and a terminator.
pMA04 construct: comprising a promoter operably linked to *gene 3, gene 4* and the *bsd* gene that confers resistance to the antibiotic blasticidin, and a terminator.
pMA05 construct: comprising a promoter operably linked to *gene 1, gene 2,* the *shble* gene that confers resistance to the antibiotic zeocin, *gene 3, gene 4* and the bsd gene that confers resistance to the antibiotic blasticidin, and a terminator.

The F2A sequence (APVKQTLNFDLLKLAGDVESNPGP; SEQ ID NO:1) is used as linker between the genes in the pMA03, pMA04 and pMA05 constructs.

*Nannochloropsis gaditana* 526 are co-transformed with the constructs pMA03 and pMA04, or transformed with the construct pMA05. Briefly, 1.10⁸ cells are collected by centrifugation at 3500xg for 13 min, washed twice in 1 ml 375 mM D-sorbitol and re-suspended in 100 µl D-sorbitol. 2µg of linearized DNA is added to the cells, kept on ice for 15 min and electroporated into 0,2 cm cuvettes, using 2400 V, 500 Ohms, 50 µF. After electroporation, cells are transferred to 5 ml of f/2 medium and incubated for 24 hours in constant light, at 100 RPM and 20°C. Cells are then pelleted (3500xg for 5min), and plated onto selective 1% agar plate containing 7 µg/ml zeocin (Zeo 7) and incubated under the same conditions. After 1 month, zeocin resistant colonies are replated onto selective 1% agar plate containing 100 µg/ml blasticidin (Bsd 100).

### Results

Transformation of pMA05 or co-transformation of pMA03+pMA04 give rise to similar number of zeocin resistant colonies, but significantly more of the zeocin-resistant pMA03+pMA04 co-transformants are also resistant to blasticidin compared to the zeocin-resistant pMA05 transformants. These data show that expression of the 6^{th} gene on a multigene cassette is less efficient than using an expression system of the present invention comprising two expression cassettes of 3 genes.

## Claims

1. A multigene expression system for simultaneous expression of at least 6 transgenes, comprising at least two different nucleic acid expression cassettes, wherein each expression cassette comprises a promoter operably linked to three or more transgenes connected to one another by at least one sequence encoding a 2A peptide, and wherein the promoters of the expression cassettes are the same.

2. The expression system according to claim 1, wherein each expression cassette comprises a promoter operably linked to three or more transgenes connected to one another by at least two successive sequences encoding a 2A peptide.

3. The expression system according to any one of claims 1 or 2, wherein each expression cassette comprises three transgenes.

4. The expression system according to any one of claims 1 to 3, wherein the 2A peptide is derived from foot-and-mouth disease virus (FMDV 2A or F2A).

5. The expression system according to claim 1 to 4, further comprising one or more nucleic acid expression cassettes comprising a selectable marker gene.

6. The expression system according to any one of claims 1 to 5, wherein the transgenes encode for enzymes involved in a biosynthetic pathway.

7. The expression system according to claim 6, wherein the transgenes encode enzymes involved in the fatty acid biosynthetic pathway.

8. A vector system comprising the expression system according to any one of claims 1 to 7, said vector system comprising at least two vectors, wherein each vector comprises one of said at least two nucleic acid expression cassettes comprising a promoter operably linked to three or more transgenes connected to one another by at least one sequence encoding a 2A peptide.

9. The vector system according to claim 8, wherein each vector further comprises a nucleic acid expression cassette comprising a selectable marker gene.

10. The vector system according to claim 8 or 9, wherein the vectors are plasmids.

11. A host cell comprising the expression system according to any one of claims 1 to 7 or the vector system according to any one of claims 8 to 10.

12. The host cell according to claim 11, wherein the host cell is a microalga, preferably a diatom such as *Phaeodactylum tricornutum,* or a *Nannochloropsis* species.

13. A method for genetically modifying a host cell with multiple genes comprising the following steps:
- providing a host cell, and
- transforming the host cell with at least two different nucleic acid expression cassettes, wherein each expression cassette comprises a promoter operably linked to three or more transgenes connected to one another by at least one sequence encoding a 2A peptide, and optionally one or more nucleic acid expression cassettes comprising a selectable marker gene.

14. The method according to claim 13, wherein the at least two nucleic acid expression cassettes and optionally the one or more nucleic acid expression cassettes comprising a selectable marker gene are co-transformed into the host cell.

15. The method according to claim 13 or 14, further comprising the step of selecting the host cells which have been transformed with said at least two nucleic acid cassettes and said one or more nucleic acid expression cassettes comprising a selectable marker gene by culturing the host cells on a selective medium, wherein the ability of a host cell to be cultured on the selective medium is dependent on the expression of the selectable marker gene.

## Patentansprüche

1. Multigenexpressionssystem zur gleichzeitigen Expression von mindestens 6 Transgenen, umfassend mindestens zwei verschiedene Nukleinsäure-Expressionskassetten, wobei jede Expressionskassette einen Promotor umfasst, der funktionsfähig an drei oder mehr Transgene gebunden ist, die durch mindestens eine Sequenz, die ein 2A-Peptid codiert, miteinander verbunden sind, und wobei die Promotoren der Expressionskassetten gleich sind.

2. Expressionssystem nach Anspruch 1, wobei jede Expressionskassette einen Promotor umfasst, der funktionsfähig an drei oder mehr Transgene gebunden ist, die durch mindestens zwei aufeinanderfolgende Sequenzen, die ein 2A-Peptid codieren, miteinander verbunden sind.

3. Expressionssystem nach einem der Ansprüche 1 oder 2, wobei jede Expressionskassette drei Transgene umfasst.

4. Expressionssystem nach einem der Ansprüche 1 bis 3, wobei das 2A-Peptid vom Maul- und-Klauenseuche-Virus (FMDV 2A oder F2A) abgeleitet ist.

5. Expressionssystem nach Anspruch 1 bis 4, ferner umfassend eine oder mehrere Nukleinsäure-Expressionskassetten, die ein selektierbares Markergen umfassen.

6. Expressionssystem nach einem der Ansprüche 1 bis 5, wobei die Transgene Enzyme codieren, die an einem Biosyntheseweg beteiligt sind.

7. Expressionssystem nach Anspruch 6, wobei die Transgene Enzyme codieren, die am Fettsäurebiosyntheseweg beteiligt sind.

8. Vektorsystem, umfassend das Expressionssystem nach einem der Ansprüche 1 bis 7, wobei das genannte Vektorsystem mindestens zwei Vektoren umfasst, wobei jeder Vektor eine der mindestens zwei Nukleinsäure-Expressionskassetten umfasst, die einen Promotor umfassen, der funktionsfähig an drei oder mehr Transgene gebunden ist, die durch mindestens eine Sequenz, die ein 2A-Peptid codiert, miteinander verbunden sind.

9. Vektorsystem nach Anspruch 8, wobei jeder Vektor ferner eine Nukleinsäure-Expressionskassette umfasst, die ein selektierbares Markergen umfasst.

10. Vektorsystem nach Anspruch 8 oder 9, wobei die Vektoren Plasmide sind.

11. Wirtszelle, umfassend das Expressionssystem nach einem der Ansprüche 1 bis 7 oder das Vektorsystem nach einem der Ansprüche 8 bis 10.

12. Wirtszelle nach Anspruch 11, wobei die Wirtszelle eine Mikroalge ist, vorzugsweise eine Kieselalge wie *Phaeodactylum tricornutum* oder eine *Nannochloropsis*-Spezies.

13. Verfahren zum genetischen Modifizieren einer Wirtszelle mit mehreren Genen, das die folgenden Schritte umfasst:
- Bereitstellen einer Wirtszelle und
- Transformieren der Wirtszelle mit mindestens zwei verschiedenen Nukleinsäure-Expressionskassetten, wobei jede Expressionskassette einen Promotor umfasst, der funktionsfähig an drei oder mehr Transgene gebunden ist, die durch mindestens eine Sequenz, die ein 2A-Peptid codiert, miteinander verbunden sind, und gegebenenfalls einer oder mehreren Nukleinsäure-Expressionskassetten, die ein selektierbares Markergen umfassen.

14. Verfahren nach Anspruch 13, wobei die mindestens zwei Nukleinsäure-Expressionskassetten und gegebenenfalls die eine oder mehreren Nukleinsäure-Expressionskassetten, die ein selektierbares Markergen umfassen, in die Wirtszelle cotransformiert werden.

15. Verfahren nach Anspruch 13 oder 14, ferner umfassend den Schritt des Selektierens der Wirtszellen, die mit den mindestens zwei Nukleinsäurekassetten und der genannten einen oder den genannten mehreren Nukleinsäure-Expressionskassetten, die ein selektierbares Markergen umfassen, transformiert wurden, durch Kultivieren der Wirtszellen auf einem selektiven Medium, wobei die Fähigkeit einer Wirtszelle, auf dem selektiven Medium kultiviert zu werden, von der Expression des selektierbaren Markergens abhängt.

## Revendications

1. Système d'expression multigénique pour l'expression simultanée d'au moins 6 transgènes, comprenant au moins deux cassettes d'expression d'acide nucléique différentes, dans lequel chaque cassette d'expression comprend un promoteur lié fonctionnellement à trois transgènes ou plus reliés les uns aux autres par au moins une séquence codant pour un peptide 2A et dans lequel les promoteurs des cassettes d'expression sont les mêmes.

2. Système d'expression selon la revendication 1, dans lequel chaque cassette d'expression comprend un promoteur lié fonctionnellement à trois transgènes ou plus reliés les uns aux autres par au moins deux séquences successives codant pour un peptide 2A.

3. Système d'expression selon l'une quelconque des revendications 1 ou 2, dans lequel chaque cassette d'expression comprend trois transgènes.

4. Système d'expression selon l'une quelconque des revendications 1 à 3, dans lequel le peptide 2A est dérivé du virus de la fièvre aphteuse (FMDV 2A ou F2A).

5. Système d'expression selon les revendications 1 à 4, comprenant en outre au moins une cassette d'expression d'acide nucléique comprenant un gène marqueur sélectionnable.

6. Système d'expression selon l'une quelconque des revendications 1 à 5, dans lequel les transgènes codent pour des enzymes impliquées dans une voie de biosynthèse.

7. Système d'expression selon la revendication 6, dans lequel les transgènes codent pour des enzymes impliquées dans la voie de biosynthèse d'acides gras.

8. Système de vecteur comprenant le système d'expression selon l'une quelconque des revendications 1 à 7, ledit système de vecteur comprenant au moins deux vecteurs, dans lequel chaque vecteur comprend l'une desdites au moins deux cassettes d'expression d'acide nucléique comprenant un promoteur lié fonctionnellement à trois transgènes ou plus reliés les uns aux autres par au moins une séquence codant pour un peptide 2A.

9. Système de vecteur selon la revendication 8, dans lequel chaque vecteur comprend en outre une cassette d'expression d'acide nucléique comprenant un gène marqueur sélectionnable.

10. Système de vecteur selon la revendication 8 ou 9, dans lequel les vecteurs sont des plasmides.

11. Cellule hôte comprenant le système d'expression selon l'une quelconque des revendications 1 à 7 ou le système de vecteur selon l'une quelconque des revendications 8 à 10.

12. Cellule hôte selon la revendication 11, dans laquelle la cellule hôte est une microalgue, de préférence une diatomée telle que *Phaeodactylum tricornutum* ou une espèce *Nannochloropsis.*

13. Procédé pour modifier génétiquement une cellule hôte avec plusieurs gènes comprenant les étapes suivantes :
- fourniture d'une cellule hôte et
- transformation de la cellule hôte avec au moins deux cassettes d'expression d'acide nucléique différentes, chaque cassette d'expression comprenant un promoteur lié fonctionnellement à trois transgènes ou plus reliés les uns aux autres par au moins une séquence codant pour un peptide 2A et éventuellement au moins une cassette d'expression d'acide nucléique comprenant un gène marqueur sélectionnable.

14. Procédé selon la revendication 13, dans lequel les au moins deux cassettes d'expression d'acide nucléique et éventuellement l'au moins une cassette d'expression d'acide nucléique comprenant un gène marqueur sélectionnable sont co-transformées dans la cellule hôte.

15. Procédé selon la revendication 13 ou 14, comprenant en outre l'étape de sélection des cellules hôtes qui ont été transformées avec lesdites au moins deux cassettes d'acide nucléique et ladite au moins une cassette d'expression d'acide nucléique comprenant un gène marqueur sélectionnable par la mise en culture des cellules hôtes sur un milieu sélectif, la capacité d'une cellule hôte à être cultivée sur le milieu sélectif étant dépendante de l'expression du gène marqueur sélectionnable.
